Europäisches Patentamt

European Patent Office

Office européen des brevets

(19)

(11) Publication number: **0 108 112**
**B1**

(12) **EUROPEAN PATENT SPECIFICATION**

(45) Date of publication of patent specification: **21.09.88**

(51) Int. Cl.⁴: **A 61 B 17/36,** A 61 F 7/00

(21) Application number: **83901592.2**

(22) Date of filing: **17.05.83**

(86) International application number:
**PCT/EP83/00124**

(87) International publication number:
**WO 83/03961 24.11.83 Gazette 83/27**

(54) **CRYOSURGICAL APPARATUS, ESPECIALLY FOR THE CRYOSURGERY OF DEEPLY LYING LESIONS.**

(30) Priority: **17.05.82 GB 8214331**

(43) Date of publication of application:
**16.05.84 Bulletin 84/20**

(45) Publication of the grant of the patent:
**21.09.88 Bulletin 88/38**

(84) Designated Contracting States:
**CH DE FR GB LI**

(56) References cited:
**EP-A-0 025 021**
**US-A-3 298 371**

(73) Proprietor: **Drukier, Andrzej Kamil**
**6 East Street**
**Winchester Massachusetts 01890 (US)**

(72) Inventor: **Drukier, Andrzej Kamil**
**6 East Street**
**Winchester Massachusetts 01890 (US)**

(74) Representative: **Manitz, Gerhart, Dipl.-Phys. Dr.**
**et al**
**MANITZ, FINSTERWALD & ROTERMUND**
**Robert-Koch-Strasse 1**
**D-8000 München 22 (DE)**

## Description

The present invention relates to cryosurgical apparatus especially for the cryosurgery of deeply lying lesions and tumors, and to a method of monitoring the progress of a cryogenic freezing process.

During the last twenty years, cryosurgery has become established as an important clinical technique. Many investigations have been carried out in a search for a better understanding of the process.

The destruction of tissues by cooling to cryogenic temperatures is usually effected with a cryoprobe. This instrument has a continuously cooled tip, the cryotip, which is placed on or into the tissue to be destroyed.

By way of background information, the state of the art in cryoprobe design will now be described briefly. Reviews of some of the most popular designs have been published elsewhere, for example, G. Garamy in "Cryosurgery", edited by R. W. Rand et al., published in 1968 by C. C. Thomas in Springfield, U.S.A.; R. F. Barron in "Cryogenics in Surgery", edited by H. von Leden et al., published by Lewis in 1971 in London, and D. E. Wild in "Practical Cryosurgery", edited by H. B. Holden and published by Pitmann 1975 in London.

There are two main types of cryoprobes currently in use:

a) high-pressure probes that use Joule-Thompson expansion to cool the tip; and

b) liquid nitrogen probes which use the latent heat of boiling nitrogen to cool the tip.

One type of liquid nitrogen cryoprobe is disclosed in US—A—3,298,371. When used in the cryosurgery of deep lying tumors, it is essential to use a cryotip of small size, i.e. cryoprobes are required with very high cooling power. However, the cooling power of high pressure probes is not as good as the cooling power of liquid nitrogen devices and only the latter are considered in the following.

Liquid nitrogen cryoprobes cool by boiling liquid nitrogen within the cryotip. It is possible to maintain a tip temperature close to the boiling point of nitrogen (−196°C at a pressure of one atmosphere) while extracting many watts of heat from the tissue. A typical liquid nitrogen system consists of:

a) an appropriate vacuum storage flask for liquid nitrogen,

b) transfer pipes and the associated valves,

c) cryoprobe having a cryotip, i.e. that part which contacts the tissue, and

d) means of controlling the cryoprobe cooling power including a device for monitoring the cryotip temperature.

Liquid nitrogen devices give rise to problems with regard to cryogenic storage transfer. The liquid is stored in the vacuum flask and its transfer to the cryoprobe is ideally through a flexible hose. The shaft of the cryoprobe usually has to be contained within an evacuated sheath to prevent the outer casing from becoming too cold and to reduce boiling of the liquid nitrogen. A few cryoprobe designs have solved the problem of liquid nitrogen transfer by incorporating a small storage dewar into the cryotip itself, or by using the alternative technique of heating the transfer tubes to maintain the liquid in Leidenfrost flow.

The cryoprobe can conveniently be considered as a shaft portion and a tip portion, i.e. the cryotip. The shaft portion is essentially the coolant transfer tube. In practice it may consist of an outer sheath, vacuum insulated from the cold, internal tube. The internal tube is subdivided into the liquid nitrogen channel and the cold gas sink. For most applications in deep-in-body cryosurgery, the cryotip should be rigid.

The cryotip is the part of the cryoprobe in which the liquid coolant is boiled off due to heat exchange with the tissue. During cryotherapy it should be placed directly into the tissue to be destroyed.

A number of theoretical studies together with measurements of the growing ice mass have afforded a reasonable understanding of the thermal effects of the cryoprobes. Monitoring the volume of the deeply frozen tissue which will be destroyed during thawing is very important if cryosurgery is to be a precise and practical medical tool. However, no satisfactory technique has so far been developed for these purposes. Usually one observes optically the development of the "ice-ball". However, this method is limited to external cryosurgery applications, for example of the skin, and is highly imprecise.

The aim of cryosurgery is to kill all cells within a well-defined region. Investigations on cell tissues frozen in vitro have indicated two main mechanisms of cell destruction, i.e. extra and intra-cellular ice formation. As the extra-cellular ice grows, cell water passes out of the cell which causes the cell to shrink. Lethal cell damage occurs on thawing due to the large uptake of water into the cell and eventual rupture.

Several authors have published theoretical models to describe the frozen mass of tissue, the "ice-ball".

When a cryotip is placed in contact with tissue and cooled a temperature gradient develops in the tissue. The tissue adjacent the probe will undergo a phase change and, as the cooling continues, the freezing front will propagate through the tissue. Eventually, a steady state will be reached where the rate at which heat is extracted by the cryoprobe balances the rate at which it is supplied by the tissue.

Cryosurgery has primary and secondary applications to diseases of the brain and spinal cord. Foremost has been its use in the production of discrete, destructive, thalamic lesions in the surgical treatment of Parkinson's disease and related disorders. Similarly, cryosurgery has been employed in the destruction of the normal pituitary gland, that is stereotactic-cryohypophysectomy, and in the treatment of selected cases of metastatic tumors. Cryosurgery is also useful in the management of difficult or unusual arterio-venous malformations of the brain and spinal cord and as an adjunct for other procedures, for example utilising the cryoprobe as a retractor in the micro-neurosurgical dissection of tumors and for the destruction of residual tumor tissues.

Cryosurgery has been used in the treatment of selected cases of metastatic tumors on the surface and in body cavities. The cryotreatment of tumours deep inside the body has been prohibited by the lack of an appropriate monitoring technique.

One of the main problems limiting the widespread use of cryosurgery is the difficulty of controlling the amount of tissue that is destroyed during the treatment. It should be pointed out that cryoprobes are a highly flexible source of cooling. The rate of cooling can be easily and precisely regulated. It has however proved very difficult to monitor the propagation of the freezing front inside the body. The classical ice-ball monitoring techniques are simply not good enough.

For the cryosurgery of surface lesions (warts, skin tumors etc.) the extent of cryodestruction is usually assessed by the extent of the white frozen area generated. This has several disadvantages, namely: the volume of tissue destroyed is somewhat less than the volume of tissue cooled below 0°C; the volume of tissue destroyed depends on the cooling speed which is difficult to assess from the extent of frost on the skin; the visual check is valid for two dimensions. The depth of deep frozen tissue can only be estimated by assuming a hemispherical ice-ball shape.

Cryoprobes have been described that are combined with endoscopes for use in cryoprostectomy, more recently, which are small enough to be introduced into the bladder or the uterus.

The most common method of monitoring ice-ball growth, other than direct visional palpation, is the use of thermocouples. They are usually mounted in hypodermic needles and are placed on the outer border of the lesion prior to cryosurgery; the freezing process is continued until the thermocouples register a temperature below zero. Practically, the usefulness of this technique depends on how accurately the thermocouples are placed. Accurate positioning can be quite difficult if the site is not accessible.

Changes in tissue impedance and of the current flowing between an active and a reference electrode placed in the frozen region have been studied. These measurements are claimed to be superior to temperature measurements because they give information about the volume of the tissue destroyed, rather than just about the volume of the ice-ball. For example the low frequency impedance rises rapidly at the eutectic point due to extracellular ice formation. However, these measurements give information only about the volume and not the shape of the ice-ball.

Although measurements of the cryoprobe tip temperature have been shown to be unreliable as an indication of ice-ball size, it has been suggested that a heat flux measurement can be used.

However, this can be used only for the initial stages of freeze.

From this brief description it can be seen that the techniques appropriate for the precise monitoring, let alone imaging of the ice-ball development are missing. Furthermore, all the classical techniques are applicable only to the cryosurgery of tissue close to the body surface.

It is a principle object of the present invention to provide improved cryosurgical apparatus for monitoring the growth of the ice-ball during cryosurgery, preferably by the use of three-dimensional imaging techniques, and, for this purpose, to provide an improved cryoprobe which makes the use of such improved imaging techniques practicable. Furthermore the present invention is also intended to provide apparatus which makes it possible for cryosurgery to be carried out at locations deep inside the body while still enabling monitoring of the cryosurgical operation.

For these purposes the invention is also concerned with the provision of a cryoprobe of reduced size and adequate cooling power, which is constructed so as to allow the use of the monitoring technique proposed herein.

According to the present invention, there is provided, starting from the prior art arrangement of US—A—3,298,371, cryosurgical apparatus comprising a cryoprobe including a hollow insulated shaft portion having first and second ends, a cryotip mounted at said first end of said shaft portion and an inner tube extending inside said hollow insulated shaft portion from said second end to said first end and defining a passage between said inner tube and said hollow insulated shaft portion; a supply of liquid cryocoolant connectable to said inner tube for supplying cryocoolant to said cryotip through said inner tube, with said cryocoolant returning through said passage; characterised by the combination with an X-ray computer tomography installation for monitoring the developing of a cryosurgical tissue freezing process carried out using said cryoprobe; and in that at least said cryotip comprises substantially exclusively, with the possible exception of a thin external layer of a noble metal, of material having a low atomic number $Z$ of less than 15.

The invention is based on the recognition that by employing a cryotip of low atomic number it will be possible to use modern methods of medical imaging namely, x-ray computer tomography, to provide a three dimensional image of the ice-ball development and indeed without the image being significantly impaired by artifacts. This apparatus offers the further advantage that the diseased tissue, for example the tumor, the position of the cryotip and the temperature distribution around the cryotip can all be visualised at the same time.

The absorption of x-rays ($E=80$ keV) in biological tissues is mainly due to Compton scattering. Thus, it is proportional to the tissue density. Using computer tomography, a density variation of a few parts per thousand can be detected. Manufacturers of CT scanners have chosen to relate attenuation specifically to water and air using a scale designed to produce a range of positive and negative integers for the materials of clinical interest, namely so-called Hausenfeld units (HU). For example, a change of density of 1% means a change of 10 HU.

Water is the main component of almost all biological tissues, for example over 90% of both blood and muscular tissue. In the temperature range of interest, i.e. from the typical body temperature of 36.6 C down to the eutectic point of the intracellular fluid, ca. −21°C, the changes of density of water are rather large. For example, the density of water is 0.9935 g/cc at 36.6 C and 0.9999 g/cc at 0°C. The difference in density, about 0.6%, is detectable with CT systems. At the freezing point the density of ice is 0.9168 g/cc, i.e., the change in density due to the change in phase is 8.3% or 83 HU. Thus, the invention is based on the realisation that computer tomography will give an image of the propagation of the freezing front and some indication of the temperature gradients involved. The inventor has proved this experimentally by in vitro studies which show that the development of the ice-ball can be easily imaged using computer tomography. Studies have been made of many tissues of biological origin; cerebrospinal fluid, blood, animal fats, meat, white and grey brain matter. These results show that the change in density due to freezing is easily detectable by computer tomography and much higher than the natural variation of density in the tissues studied. Furthermore, the data derived to date suggest that, as expected, the largest part of the density change is due to phase change.

The spatial resolution of modern CT installations is ca. 2 to 3 mm and the "slice thickness" is a few millimeters. The minimum time for reconstruction is a few seconds per slice. Thus, both the spatial and temporal resolutions of avilable CT systems are appropriate for imaging the growth of the ice-ball during cryosurgery. Real time imaging should permit feedback optimisation of cryosurgery procedures. Furthermore, cryosurgery of even the deepest internal organs becomes possible.

As previously mentioned, the invention proposes the use of a cryotip of a material of low atomic number $Z < 15$.

A cryode used for cryosurgery of the body surface, e.g. of the skin, is admittedly known from EP—A—25021 which has a cryotip of aluminum. However, this cryode is in no way adapted for use for deep-in-body cryosurgery, as can be seen simply from the fact that it is very short and of relatively large diameter, so that it cannot be used to penetrate deeply in body tissue.

Moreover, the cryode of EP—A—25021 is cooled by the Joule-Thompson effect, i.e. *expansion of gas*. For applications in cryosurgery deep in the body one however requires much higher cooling power, which is obtained in the present apparatus by the use of a liquid cryocoolant which extracts heat by evaporation inside the cryotip. EP—A—25021 does not in any way suggest the monitoring of the ice-ball via X-ray computer tomography.

The material will, for practical reasons, normally be metal and indeed one of the group comprising lithium, beryllium, magnesium and aluminium or an alloy of any of these elements, either with themselves or with other elements.

In a particularly practical embodiment the cryotip consists of an inner heat exchanger and a thin outer shell. The heat exchanger conveniently consists of a sintered metal.

In this arrangement the shell is made of aluminium or another metal of low atomic number.

In arrangements of this kind the outer shell is conveniently covered with a layer of noble metal, copper or chromium, having a thickness less than 0.5 mm and preferably less than 0.2 mm. This arrangement is not only relatively easy to manufacture but it also avoids corrosion of the outer shell and possible danger to the patient through the use of, for example beryllium.

The aforementioned layer covering the outer shell is conveniently applied by plating.

In an arrangement of the above-mentioned kind, the shaft portion preferably has a smaller cross-section than the cryotip. This arrangement is beneficial because it means that the shaft portion is less noticable in the image produced by computer tomography. In other words, the danger of creating artifacts is reduced, particularly at radiographically unfavourable angles of the cryoprobe which may be necessary in practice. The use of a shaft portion with a smaller cross-section than the cryotip is favourable in cases of deep-in-body surgery.

In practice the invention envisages a cryoprobe in which the cryotip has a cross-sectional area smaller than 20 square mm and preferably smaller than 10 square mm. The cryotip will be preferably used with a shaft portion having a cross sectional area below 20 square mm.

The cryotip is preferably egg-shaped with a relatively pointed front end which facilitates the introduction into body tissue for deep-in-body surgery. The pointed head allows the tissues to be pushed aside without being severed so that the damage caused to the body by the introduction of the cryotip is minimal. As the shaft in any case has a smaller diameter than the cryotip, the presence of the shaft is not objectionable.

One particularly advantageous arrangement, which can also be used with conventional cryoprobes, comprises the use of a coolant comprising one of liquefied nitrogen at a pressure greater than two atmospheres, a liquefied noble gas, or a mixture of a liquefied noble gas with another cryocoolant. In one particularly advantageous arrangement the liquefied noble gas is helium kept in superfluid state i.e. below 2.15 K.

An especially beneficial arrangement comprises the use of crypton or xenon or a mixture of these gases as the liquefied noble gas. This arrangement is particularly beneficial with a cryoprobe of small dimensions, in accordance with the present teaching, because it can be transferred through the relatively narrow shaft portion to the cryotip without suffering an undue pressure loss and can develop the maximum cooling power in the cryotip for a given size thereof.

A further problem which is encountered with existing cryoprobes, and which becomes more critical as the shaft portion is made smaller in cross-section, is the problem of preventing the outside of the shaft portion from becoming too cool. In the case of deep-in-body cryosurgery, this cooling may cause damage of the tissue surrounding the shaft portion. Conventional cryoprobes use a vacuum jacket to prevent the cooling of the outside of the shaft portion. The use of a vacuum jacket becomes however increasingly difficult as the diameter of the shaft portion reduces. In order to overcome this difficulty the present invention proposes the arrangement set forth in Claims 7 to 14.

According to a particularly favourable aspect of the invention there is provided, starting from the prior art of US—A—3,298,371 cryosurgical apparatus including a cryoprobe comprising an elongate shaft portion, a cryotip at one end of said shaft portion, a first passage defined within said shaft portion for conveying a liquid cryocoolant to the inside of the cryotip to cool the same, a second passage defined within said shaft for return flow of the cryocoolant, and means for heating said elongate shaft portion; characterised in that the external diameter of said elongate shaft portion is substantially the same size as or smaller than the external diameter of said cryotip; in that at least the cryotip consists of a material or materials of low atomic number $Z \leq 15$, with the possible exception of a thin external layer of a noble metal; and in that the means for heating said shaft portion comprises a tubular structure defining an optical cavity having a reflective inner surface and an absorptive outer surface, and means for introducing light energy into said optical cavity.

An additional problem which arises in cryosurgery is the difficulty of extracting the cryoprobe from the frozen tissue. It will be appreciated that the cryoprobe can only be withdrawn once the bond to the ice-ball has been broken. Release of the cryoprobe from the ice-ball can be achieved by heating the tip. A conventional method involves Joule heating of bulk resistor placed in the tip and is ·considered undesirable as the resistor will probably cause artifacts in the image. In order to overcome this difficulty, the present invention proposes the arrangements set forth in Claims 15 and 16.

Finally, the present invention also relates to a method of monitoring the progress of a cryogenic freezing process, other than a method of treating the human or animal body, comprising the use of x-ray computer tomography to visualise phase changes through the associated density changes.

Embodiments of the invention will now be described by way of example and with reference to the drawings which show:

Figure 1, a schematic illustration of a basic cryoprobe unit for cryotherapy, and

Figure 2, a longitudinal section through a cryotip.

Figure 3, a longitudinal section through an alternative cryoprobe with provision for heating the shaft portion thereof,

Figure 4, a longitudinal section through a further alternative cryoprobe also with provision for heating the shaft portion thereof,

Figure 5, a longitudinal section through a yet further alternative cryoprobe also with provision for heating the shaft portion thereof,

Figure 6, a longitudinal section through another cryoprobe with provision for heating the cryotip, and,

Figure 7, a longitudinal section through a cryoprobe similar to that of Figure 6 but with an alternative arrangement for heating the cryotip.

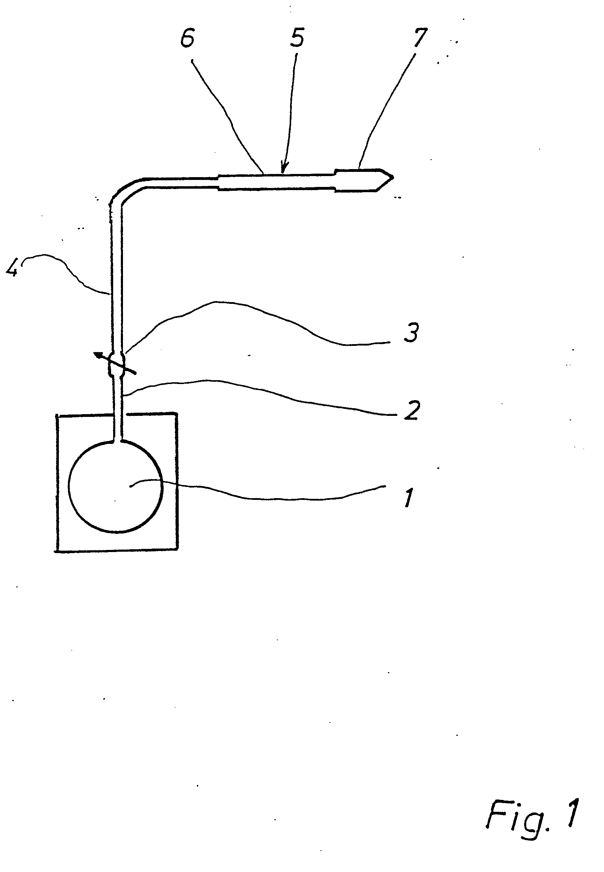

Referring firstly to Figure 1, there can be seen a source 1 of a liquefied gas, which can be liquid nitrogen at a pressure of more than 2 atmospheres (and preferably more than 5 atmospheres) a liquified noble gas or a miture of a liquefied noble gas with another cryocoolant. Source 1 is connected via a line 2 and an adjustable valve 3 to a flexible hose 4 leading to a cryoprobe 5. In accordance with the invention the liquefied gas is preferably liquefied xenon which, as previously explained, will result in the maximum cooling power in a cryoprobe of the presently described kind.

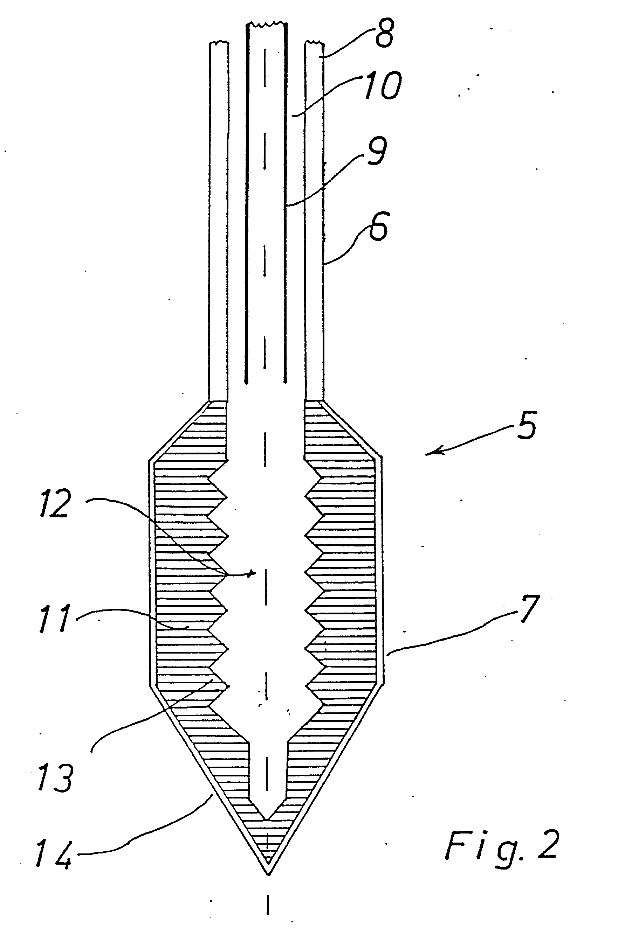

As illustrated schematically in Figure 1, and in more detail in Figure 2, the cryoprobe 5 comprises a shaft portion 6 and a cryotip 7. The shaft portion 6 serves to transfer the liquefied noble gas to the cryotip 7. For this purpose, the shaft portion comprises an inner tube 9 through which the liquefied gas enters the head portion 7. The cryotip 7 consists of a sintered metal body part 11 of a metal of low atomic number $Z < 15$ and indeed preferably of aluminium. A threaded passage 12 is provided inside the sintered body portion 11 so as to provide a large surface area for the transfer of heat from the sintered body portion to the cryocoolant. It will be appreciated that the rate of heat transfer is improved by the large surface area offered by the flanks of the threads 13. At its front end, the body portion 11 converges to a sharp point which facilitates its introduction into body tissue.

As previously mentioned, the sintered body portion is enclosed by a thin plated layer 14 of an inert metal which is also easy to sterilize. The inert metal is in this case gold, the large atomic number of gold is not however disadvantageous because the gold layer is very thin in comparison to the sintered body portion.

After the liquefied gas or cryocoolant has entered the threaded passage 12 of the body portion 11 a phase change occurs and the gas that is generated is extracted through the coaxial passage 10 defined between the inner tube 9 and the tubular wall structure 8 of the shaft portion.

This gas is collected for re-use either through a line running parallel to the flexible hose or through an outer annular passage coaxial to the feed passage through the flexible hose.

The maximum diameter of the generally egg-shaped head portion 7 is approximately 5 mm, thus generating a cross-sectional area of the order of 20 square mm. The shaft portion is of smaller dimensions and preferably has an external diameter such that its cross-section is a maximum of 20 square mm and preferably 10 square mm. The threaded passage 12 will have a major diameter of, at the most, 2 mm and the remaining dimensions will be approximately as shown in the scale drawing of Figure 2.

In a particularly preferred arrangement not shown the heat exchanger is formed of a sintered metal such as aluminium (the sintered material has an even lower density than the straight metal), the heat exchanger is coated with a layer of aluminium to form a continuous outer shell which is substantially impermeable and this outer shell is then coated e.g. by plating, with a layer of gold which is substantially inert as far as body fluids are concerned. The outer shell can be formed by electroplating or using other known techniques.

The cryotip can of course also be formed in a single piece of metal (plated if necessary).

It will be appreciated in all the above embodiments that the tubular wall structure 8 is a conventional vacuum wall structure i.e. comprises tubular inner and outer metallic walls separated by a vacuum cavity.

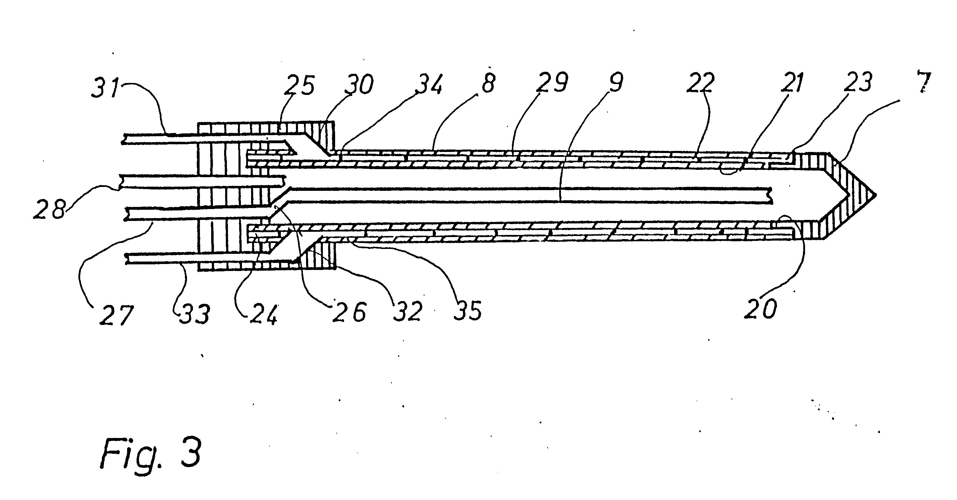

Returning now to Figure 3, there can be seen an alternative design of cryoprobe, the length of the shaft has however been considerably shortened for the purpose of illustration. In this case the cryotip 5 consists of a piece of solid aluminium which has been turned to provide an annular shoulder 20. This shoulder serves for attachment of the cryotip to the tubular wall structure 8 which, in this case, consists of plastic material, for example polyvinylchloride or PTFE. The tubular wall structure 8 comprises an inner tubular wall member 21 and an outer tubular wall member 22 which are spaced apart by two plastic spacers 23 and 24 at the axial ends of the tubular wall structure. The annular spacers 23 and 24 are secured to the inner and outer tubular members 21 and 22 by adhesive or by a plastic welding technique. The resulting tubular wall structure is secured to the cryotip at the shoulder either by means of adhesive or preferably by means of a fine screw thread (not shown) and a blocking adhesive. The end of the tubular structure remote from the cryotip is embedded in a block of plastic 25 which contains a number of passages and drillings which will now be explained in more detail.

As can be seen the inner tube 9 which is preferably a thin-walled aluminium tube includes a staggered portion 26 and a straight portion 27 which extends through the plastic block 25 and which provides the inlet connection for the supply of liquid cryocoolant. Immediately above the tubular portion 27 there is illustrated a second metallic thin walled tube 28 which extends parallel to the tube 27 through a passage in the plastic block 25 and which forms the outlet for the cryocoolant after the latter has passed through the cryotip 5.

As is clear from the drawing, the inner and outer tubular wall members are spaced apart by the spacers 23, 24 to define a cavity 29. A cross-drilling 30 passes through the plastic block and through the tubular outer wall member to provide a passage communicating with the cavity 29. A tubular insert 31 extending parallel to the tube portions 27 and 28 breaks into the passage 30, which is plugged at its outer end, and provides an inlet for supplying a heating fluid to the cavity 29. A similar cross-drilling 32 and tubular insert 33 is provided for removing the heating fluid from the cavity 29. The cavity 29 includes two helical coils of plastic strip 34, 35, in the form of a two start thread. These helical strips are so arranged that the heating fluid is caused to flow in a first helical path through the cavity 29 to the cryotip and back along a second parallel helical path to the outlet 33. In place of the two helical coils 34 and 35, one can also use two partition walls extending in an axial direction.

In practice, a heating fluid, for example hot air is supplied to the passage 29 in order to prevent freezing at the outer surface of the tubular wall structure 8. In practice it is generally not necessary to use this heating system until some time after an operation has commenced because it has been found that a considerable time is required for the outer wall surface of the tubular wall structure 8 to reach a temperature at which freezing of the surrounding tissues occurs. In fact, for some applications, it may not be necessary to use a heating fluid at all and in this case the cavity can conveniently be filled with a getter for residual gas such as activated carbon, and the tubes 31 and 33 sealed off, possibly after having effected a partial evacuation of the cavity 29. One thus has a type of vacuum insulated structure which further impedes the cooling of the outer surface of the tubular wall structure 8.

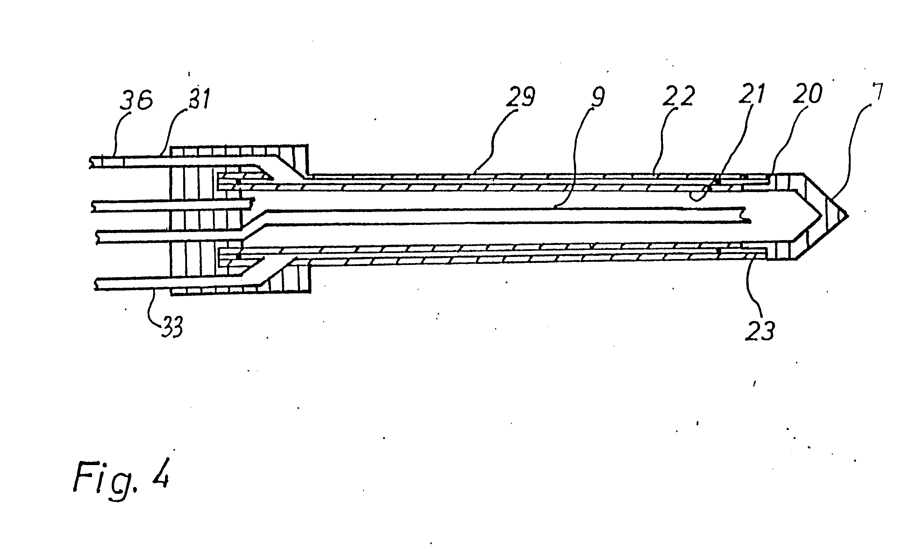

A similar arrangement to that shown in Figure 3 is illustrated in Figure 4. The same reference numerals will be used in Figure 4 to designate parts which have counterparts in Figure 3. In the arrangement of Figure 4 the cavity 29 is designed to accept a chemical or chemicals which generate heat exothermically. In this case the chemicals, which are preferably two liquids, are injected through the passage 31 using a hypodermic syringe at the start of the operation. The passage 31 is conveniently closed by a removable self-sealing rubber bung 36. The two chemicals can be selected so that the exothermic generation of heat takes place at an increasing rate so that the shaft portion is progressively heated as the operation takes place thus compensating for the otherwise decreasing temperature of the outer surface of the tubular wall structure 8. The second tube 33 can be used to extract the liquids after the reaction so that the cryoprobe can be prepared for re-use. Alternately one chemical can be introduced through the tube 31 and the second chemical through the tube 33 in order to prevent mixing of the two chemicals prior to insertion into the cryoprobe. In a further alternative version of this arrangement the outer wall member 22 consists of a thin metal tube and a single chemical in the form of an acid is introduced into the cavity through the tubular insert 31. The acid is selected to exothermically etch away part of the inside of the tubular wall during the operation thus generating sufficient heat to prevent freezing of the tissue surrounding the shaft portion.

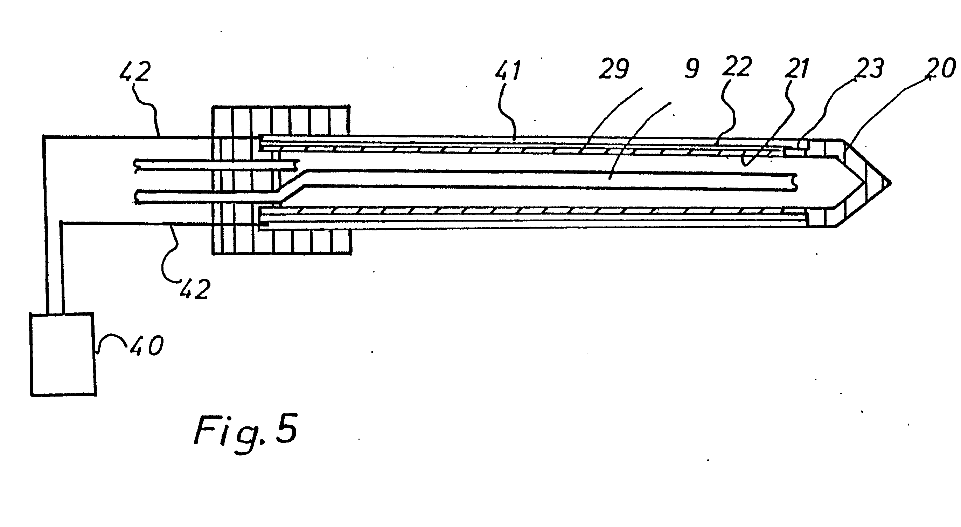

A further alternative embodiment in which provision is made for heating the outer surface of the shaft portion 8 is illustrated in Figure 5. Again common reference numerals are used to indicate parts which have counterparts in the previous embodiments. In the arrangement of Figure 5, the outer tubular wall member consists of a translucent and preferably transparent plastic which is silvered at its inner surface to make it light reflecting and which is provided with an absorptive outer surface for example by blackening the outer surface. A laser light source 40 is connected to the resulting optical cavity 41 via a plurality of optical fibres 42 of which only two are shown in Figure 5 for the sake of simplicity. Thermal energy in the form of light generated by the laser 40 is thus introduced into the optical cavity 41 and the light is progressively reflected from the silvered inner surface of the cavity 41 to the blackened outer surface where it is absorbed thus preferentially generating heating at the outer surface. In this embodiment the tubular wall structure again features inner and outer tubular wall members 21 and 22 separated by a small cavity 29. It is however conceivable that this embodiment will work with solely a single tubular member due to the preferential heating of the outer surface.

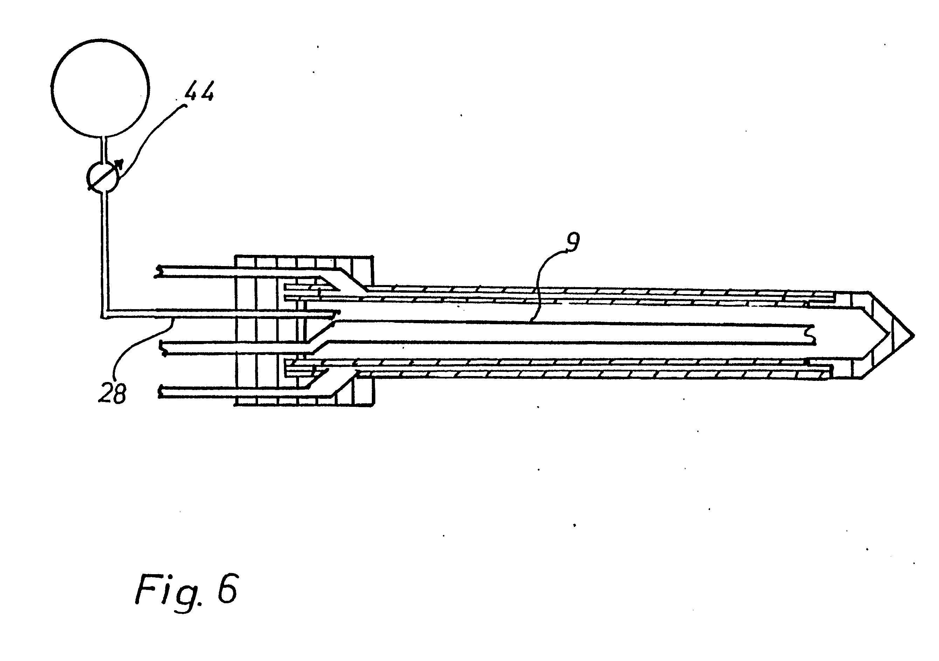

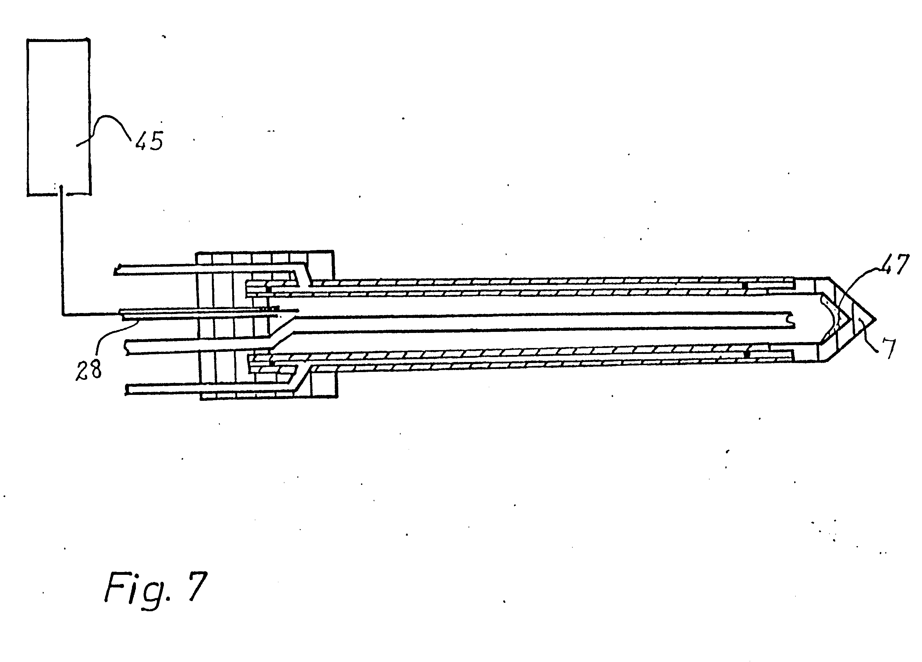

Turning now to Figure 6 there can be seen an alternative design of cryoprobe which has provision for heating the cryotip after completion of the freezing phase so as to melt a layer of water around the tip and to facilitate its withdrawal from the patient. In this embodiment a source of heated gas, for example hot air, can be connected via a valve 44, to the tubular insert 28 normally used for removal of the cryocoolant from the cryoprobe. In this case the tube 9 acts as the outlet for the heating fluid. Clearly suitable valves are required in the inlet and outlet flow lines to enable the surgeon to select heating or cooling as required. In an alternative arrangement, Figure 7, a laser light source 45 may be used to heat the cryotip. For this purpose a light guide, for example in the form of a fibre or a bundle of optical fibres, extend from the laser through the tubular insert 28, or through a second specially provided tubular insert (not shown). Light from the laser is directed towards the cryotip which may be provided with a blackened surface 47 so that it is strongly absorbent. If the arrangement of Figure 7 is used with an arrangement similar to Figure 5, then the laser light source 40 can be used in place of the laser light source 45.

In all the embodiments of Figures 3 through 7 the cryotip is only schematically illustrated and it will be appreciated that the tip can, if desired, be made larger than the shaft and can, optionally, be constructed in the same manner as the cryotip of Figure 2.

It will. be appreciated by the person averagely skilled in the art that x-ray computer tomography can readily be used to monitor the development of the ice ball during use of any of the above-described cryoprobes for deep in-body surgery without the presence of the cryoprobe causing artefacts which confuse the radiographic image.

## Claims

1. Cryosurgical apparatus comprising

a cryoprobe (5) including a hollow insulated shaft portion (6) having first and second ends, a cryotip (7) mounted at said first end of said shaft portion (6) and an inner tube (9) extending inside said hollow insulated shaft portion from said second end to said first end and defining a passage (10) between said inner tube (9) and said hollow insulated shaft portion (6);

a supply (1) of liquid cryocoolant connectable to said inner tube (9) for supplying cryocoolant to said cryotip (7) through said inner tube (9), with said cryocoolant returning through said passage (10);

characterised by the combination with an X-ray computer tomography installation for monitoring the development of a cryosurgical tissue freezing process carried out using said cryoprobe (5); and in that at least said cryotip (7) comprises substantially exclusively, with the possible exception of a thin external layer of a noble metal, of material having a low atomic number Z of less than 15.

2. Cryosurgical apparatus in accordance with Claim 1, wherein said material is a metal, in particular an element of the group comprising Li, Be, Mg, and aluminium, or an alloy of any of these elements, either with themselves or with other elements.

3. Cryosurgical apparatus in accordance with either of the preceding claims, wherein the cryotip (7) consists of an inner heat exchanger (11) and a thin outer shell (19), and wherein the heat exchanger (11) preferably consists of a sintered metal.

4. Cryosurgical apparatus in accordance with Claim 3, wherein the shell (14) is made of aluminium or another metal of low atomic number, and is preferably covered with a layer of noble metal, copper or chromium having a thickness less than 0.5 mm and preferably less than 0.2 mm, said layer being preferably applied by plating.

5. Cryosurgical apparatus in accordance with any one of the preceding claims, wherein the shaft portion (6) has a smaller cross-section than the cryotip (7), said cryotip (7) preferably having a cross-sectional area smaller than 20 square mms and in particular less than 10 square mms.

6. Cryosurgical apparatus in accordance with any one of the preceding claims, wherein said cryotip (5) is substantially egg-shaped and/or is provided with a sharp point at its front end.

7. Cryosurgical apparatus in accordance with one of the preceding claims, wherein the coolant comprises one of liquefied nitrogen at a pressure greater than two atmospheres, a liquefied noble gas or a mixture of a liquefied noble gas with another cryocoolant, and wherein said liquefied noble gas is preferably either helium kept in superfluid state, i.e. below 2.15 K, or krypton or xenon or a mixture thereof.

8. Cryosurgical apparatus in accordance with any one of the preceding claims wherein the shaft portion (6) comprises an inner tube (9) positioned within a tubular wall structure, said tubular wall structure having substantially concentrically disposed inner and outer tubular wall members (21, 22).

9. Cryosurgical apparatus in accordance with Claim 8, wherein said inner and outer tubular wall members (21, 22) are spaced apart by a cavity (29), which may contain a getter for gas such as activated carbon.

10. Cryosurgical apparatus in accordance with Claim 9, wherein means (31) are provided for supplying a heating fluid to said cavity (29) at an inlet end thereof remote from said cryotip (7), wherein means (34, 35) are provided in said cavity for causing said heating fluid to flow along said cavity (29) to said cryotip (7) and back to said inlet end, and wherein means (33) are provided at said inlet end for removing said heating fluid from said cavity.

11. Cryosurgical apparatus in accordance with Claim 10, wherein said means for causing said heating fluid to flow along said cavity (29) comprises a helical structure (34, 35) or tube disposed within said cavity, or partition wall means extending along said cavity to said cryotip.

12. Cryosurgical apparatus in accordance with Claim 9, wherein means (31, 36) is provided for introducing a chemical or chemicals into said cavity to generate heat exothermically.

13. Cryosurgical apparatus in accordance with Claim 1 or Claim 8, wherein the outer tubular wall member (22) comprises a translucent material and has a reflective internal surface and an absorptive outer surface, and wherein means (40, 42) is provided for introducing light energy into said outer tubular wall member, said means preferably comprising a fibre optical system (42).

14. Cryosurgical apparatus in accordance with any one of the Claims 8 to 13, wherein said inner and outer tubular members (21, 22) are of synthetic material.

15. Cryosurgical apparatus in accordance with Claim 1 or Claim 8 and wherein means (28) are provided for supplying a heating fluid to said cryotip (7) via either said inner tube (9) or via the space (10) between the inner tube (9) and the tubular wall structure (21, 22).

16. Cryosurgical apparatus in accordance with Claim 1 or Claim 8, wherein means (45) is provided for directing laser light through said inner tube (9) or through the space (10) between the inner tube (9) and the tubular wall structure (21, 22) to said cryotip (7) to heat the same.

17. Cryosurgical apparatus including a cryoprobe (5) comprising an elongate shaft portion (6), a cryotip (7) at one end of said shaft portion (6), a first passage defined within said shaft portion for conveying a liquid cryocoolant to the inside of the cryotip (7) to cool the same, a second passage defined within said shaft portion for return flow of the cryocoolant, and means (40, 42) for heating said elongate shaft portion (6);

characterised in that the external diameter of said elongate shaft portion (6) is substantially the same size as or smaller than the external diameter of said cryotip (7); in that at least the cryotip (7) consists of a material or materials of low atomic number $Z \leq 15$, with the possible exception of a thin external layer of a noble metal; and in that the means for heating said shaft portion comprises a tubular structure (22) defining an optical cavity (41) having a reflective inner surface and an absorptive outer surface, and means (40, 42) for introducing light energy into said optical cavity.

18. A method of monitoring the progress of a cryogenic freezing process, other than a method of treating the human or animal body, comprising the use of X-ray computer tomography to visualise phase changes through the associated density changes.

**Patentansprüche**

1. Kryo-chirurgische Vorrichtung mit einer Kryo-Sonde (5), die einen hohlen isolierten Schaftabschnitt (6) mit ersten und zweiten Enden enthält, eine an dem ersten Ende des Schaftabschnittes (6) angebrachte Kyro-Spitze (7) und ein Innenrohr (9), das sich in dem hohlen isolierten Schaftabschnitt von dem zweiten Ende bis zum ersten Ende erstreckt und einen Durchlaß (10) zwischen dem Innenrohr (9) und dem hohlen isolierten Schaftabschnitt (6) bestimmt;

einem Vorrat (1) aus flüssigem Kryo-Kühlmittel, der mit dem Innenrohr (9) zum Zuführen von Kryo-Kühlmittel zu der Kryo-Spitze (7) durch das Innenrohr (9) verbindbar ist, wobei das Kryo-Kühlmittel durch den Durchlaß (10) zurückkehrt;

gekennzeichnet durch die Kombination mit einer Röntgen-Computer-Tomografie-Einrichtung zum Überwachen der Entwicklung eines unter Benutzung der Kryo-Sonde (5) ausgeführten kryochirurgischen Gewebefriervorganges; und

dadurch, daß mindestens die Kryo-Spitze (7) im wesentlichen ausschließlich aus einem Edelmetall aus Material mit einer niedrigen Atomzahl $Z < 15$ besteht, mit der möglichen Ausnahme einer dünnen Außenschicht.

2. Kyro-chirurgische Vorrichtung nach Anspruch 1, bei der das Material ein Metall, insbesondere ein Element der aus Li, Be, Mg und Aluminium oder einer Legierung aus irgendwelchen dieser Elemente entweder mit denselben oder mit anderen Elementen bestehenden Gruppe ist.

3. Kyro-chirurgische Vorrichtung nach einem der vorangehenden Ansprüche, bei der die Kyro-Spitze (7) aus einem inneren Wärmetauscher (11) und einen dünnen Außenmantel (19) besteht und bei der der Wärmetauscher (11) vorzugsweise aus einem Sintermetall besteht.

4. Kryo-chirurgische Vorrichtung nach Anspruch 3, bei der der Mantel (14) aus Aluminium oder einem anderen Metall mit niedriger Atomzahl gefertigt ist und vorzugsweise mit einer Schicht aus Edelmetall, Kupfur oder Chrom mit einer Stärke von weniger als 0,5 mm und vorzugsweise weniger als 0,2 mm bedeckt ist, wobei die Schicht vorzugsweise durch plattieren aufgebracht ist.

5. Kryo-chirurgische Vorrichtung nach einem der vorangehenden Ansprüche, bei der der Schaftabschnitt (6) einen kleineren Querschnitt als die Kyro-Spitze (7) besitzt, wobei die Kyro-Spitze (7) vorzugsweise eine Querschnittsfläche von weniger als 20 mm² und insbesondere weniger als 10 mm² besitzt.

6. Kryo-chirurgische Vorrichtung nach einem der vorangehenden Ansprüche, bei der die Kryo-Spitze (5) im wesentlichen eiförmig und/oder mit einer scharfen Spitze an ihrem vorderen Ende versehen ist.

7. Kryo-chirurgische Vorrichtung nach einem der vorangehenden Ansprüche, bei der das Kühlmittel aus einem der Stoffe verflüssigter Stickstoff bis einem Druck von über 2 Atmosphären, ein verflüssigtes Edelgas oder ein Gemisch aus einem verflüssigten Edelgas mit einem anderen Kryo-Kühlmittel ist und bei dem das verflüssigte Edelgas vorzugsweise entweder in superfluidem Zustand, d. h. unter 2,5 K gehaltenes Helium oder Krypton oder Xenon oder ein Gemisch daraus ist.

8. Kyro-chirurgische Vorrichtung nach einem der vorangehenden Ansprüche, bei der der Schaftabschnitt (6) ein in eine rohrförmige Wandstruktur eingesetztes Innenrohr (9) umfaßt, wobei die rohrförmige Wandstruktur im wesentlichen konzentrisch angeordnete Innen- und Außen-Rohrwandelemente (21, 22) besitzt.

9. Kryo-chirurgische Vorrichtung nach Anspruch 8, bei der die inneren und äußeren Rohrwandelemente (21, 22) durch einen Hohlraum (29) voneinander getrennt sind, der einen Gasgetter wie Aktivkohle enthalten kann.

10. Kryo-chirurgische Vorrichtung nach Anspruch 9, bei der Mittel (31) vorgesehen sind zum Zuführen eines Heizfluides zu dem Hohlraum (29) an einem von der Kryo-Spitze (7) abgelegenen Einlaßende derselben, wobei Mittel (34, 35) in dem Hohlraum vorgesehen sind, um das Heizfluid längs des Hohlraumes (29) zu der Kryo-Spitze (7) und zurück zum Einlaßende strömen zu lassen, und bei der an dem Einlaßende Mittel (33) zum Entfernen des Heizfluides aus dem Hohlraum vorgesehen sind.

11. Kryo-chirurgische Vorrichtung nach Anspruch 10, bei der die Mittel, um das Heizfluud längs des Hohlraumes (29) strömen zu lassen, eine in dem Hohlraum angeordnete Wendelstruktur (34, 35) oder Röhre, oder Teilwandmittel umfaßt, die sich längs des Hohlraumes zu der Kryo-Spitze erstrecken.

12. Kryo-chirurgische Vorrichtung nach Anspruch 9, bei der Mittel (31, 36) zum Einführen einer Chemikalie oder von Chemikalien in den Hohlraum vorgesehen sind, um exothermische Wärme zu erzeugen.

13. Kryo-chirurgische Vorrichtung nach Anspruch 1 oder Anspruch 8, bei der das äußere Rohrwandelement (22) ein durchscheinen des Material enthält und eine reflektierende Innenfläche und eine absorbierende Außenfläche besitzt, und bei der Mittel (40, 42) vorgesehen sind zur Einführung von Lichtenergie in das äußere Rohrwandelement, wobei die Mittel vorzugsweise ein faseroptisches System (42) umfassen.

14. Kryo-chirurgische Vorrichtung nach einem der Ansprüche 8 bis 13, bei der die Innen- und außen-Rohrelemente (21, 22) aus synthetischem Material bestehen.

15. Kryo-chirurgische Vorrichtung nach Anspruch 1 oder Anspruch 8, und bei der Mittel (28) vorgesehen sind zum Zuführen eines Heizfluides zu der Kryo-Spitze (7) über entweder das Innenrohr (9) oder über den Raum (10) zwischen dem Innenrohr (9) und der rohrförmigen Wandstruktur (21, 22).

16. Kryo-chirurgische Vorrichtung nach Anspruch 1 oder Anspruch 8, bei der Mittel (25) zum Richten von Laserlicht durch das Innenrohr (9) oder durch den Raum (10) zwischen dem Innenrohr (9) und der Rohrwandstruktur (21, 22) zu der Kryo-Spitze (7) vorgesehen sind, um diese zu erhitzen.

17. Kryo-chirurgische Vorrichtung einschließlich einer Kryo-Sonde (5) mit einem länglichen Schaftabschnitt (6), einer Kryo-Spitze (7) an einem Ende des Schaftabschnittes (6), einem ersten in dem Schaftabschnitt bestimmten Durchlaß zum Fördern eines flüssigen Kryo-Kühlmittels zu der Innenseite der Kryo-Spitze (7), um diese zu kühlen, einem zweiten innerhalb des Schaftabschnitts bestimmten Durchlaß zur Rückströmung des Kryo-Kühlmittels und Mitteln (40, 42) zum Heizen des länglichen Schaftabschnittes (6), dadurch gekennzeichnet, daß der Außendurchmesser des länglichen Schaftabschnittes (6) im wesentlichen von gleicher Größe wie oder kleiner als der Außendurchmesser der Kryo-Spitze (7) ist, daß mindestens die Kryo-Spitze (7) aus einem Material oder aus Materialien mit niedriger Atomzahl Z≤15 besteht, mit der möglichen Ausnahme einer dünnen Außenschicht aus einem Edelmetall, und daß die Mittel zum Heizen des Schaftabschnittes eine Rohrstruktur (22) umfassen, die einen optischen Hohlraum (41) mit einer reflektierenden Innenfläche und einer absorbierenden Außenfläche bestimmt, und Mittel (40, 42) zum Einführen von Lichtenergie in den optischen Hohlraum.

18. Verfahren zum Überwachen des Fortgangs eines kryogenen Gefriervorganges außer einem Verfahren des Behandelns eines menschlichen oder tierischen Körpers, mit Verwendung von Röntgen-Computertomografie zur Sichtbarmachung von Phasenänderungen durch die zugehörigen Dichteänderungen.

**Revendications**

1. Dispositif destiné à des traitements de cryochirurgie (5) comprenant une partie cylindrique (6) terminée par une partie conique dite cryotip (7) et comprenant un tube isolé (9) qui définie une voie de passage (10) entre le réservoir (1) de cryo-liquid et la dite cryotip (7) et qui est modifié pour l'opération sous controle d'un appareillage tomographique à rayon X, c'est à dire que la dite cryotip (7) consiste essentialement de materiaux à nombre atomique Z plus petit que 15.

2. Dispositif selon la revendication 1, caractérisé par le fait que les dits materiaux à bas Z sont metaux et plus particulierement Li, Be, Mg et Al ou les alliage de ces éléments.

3. Dispositif selon revendications 1 et 2, caractérise par le fait que cryotip (7) est un échangeur de chaleur (11) constitue d'un poudre métallique comprimée couvert par une couche mince métallique (14).

4. Dispositif selon revendication 3, caractérise par le fait que la dite couche métallique (14) consiste d'aluminium ou d'un autre métal a bas nombre atomique, et est couverte d'une couche additionnelle très mince (préférablement d'une epaisseur inférieur à 0.2 mm) d'un metal noble ou couivre ou chromium.

5. Dispositif selon les revendications precedentes, caractérisé par le fait que la partie cylindrique (5) est d'un diamètre inférieur au diamètre de la cryotip (7), la dite cryotip ayant un diamère plus petit que 0.5 cm.

6. Dispositif selon les revendications précedentes, caractérisé par le fait que la dite cryotip (5) est une ellipsoide avec une partie frontalière conique ou pointu.

7. Dispositif selon les revendications précedentes, caractérisé par le fait que le cryo-liquid est l'azote liquide sous pression supérieur a 2 Atm, ou un gas noble liquifié, e.g., Hélium (preferablement sur liquide), Krypton ou Xénon.

8. Dispositif selon les revendications précedentes, caractérisé par le fait que le partie cylindrique (6) inclus un tube intérieur (9) et deux tubes concentriques (21, 22).

9. Dispositif selon la revendication 8, caractérisé par le fait que l'espace (29) entre les dits tubes concentriques (21, 22) contient un material actif, e.g. charbon absorbant.

10. Dispositif selon la revendication 9, caractérisé par le fait que le dit espace (29) et la cryotip (7) sont chauffe avec un liquid circulant dans les tubes (34, 35) et evecue par le passage (33).

11. Dispositif selon la revendication 10, caractérisé par le fait que le dit espace (29) inclus une structure en hélice (34, 35).

12. Dispositif selon la revendication 9, caractérisé par le fait que les dit produits chimiques circules dans le passages (31, 36) et dépose la chaleur par réaction exothermique.

13. Dispositif selon les revendications 1 ou 8, caractérisé par le fait que le dit tube (22) est transparent avec surface extérieur absorbante et surface intérieur réfléchisante et inclue un conduit optique (40, 42) (preferablement à fibre optique) qui permet l'illumination de l'intérieur de dispositif.

14. Dispositif selon une de revendication 8, 9, 10, 11, 12 et/ou 13, caractérisé par le fait que les tubes (21, 22) sont construit de plastique.

15. Dispositif selon les revendications 1 et 8, caractérisé par le fait que un passage (28) est introduit dans la cryotip (7) permettant le transfer d'une liquide chauffe via tube (9) ou espace (10) entre la tube (9) et les tubes (21, 22).

16. Dispositif selon les revendications 1 ou 8, caractérisé par le fait que un passage (45) est introduit dans le cryotip (7) permetant l'illumination par laser via tube (9) ou l'espace (10) entre la tube (9) et les tubes (21, 22).

17. Dispositif destine a des traitements de cryochirurgie (5) comprenant une partie cylindrique (6) terminée par la cryotip (7), caractérisé par les faites suivante:—

la cryotip (7) est d'un diametre superieur a partie cylindrique (6);

cryoliquide circule dans la cryotip (7) construit de matériaux a bas nombre atomique (Z⩽15);

la chauffage de partie cylindrique (6) est permit par illumination de structure tubular (22) qui est une cavite optique (41) avec surface extérieur reflechisant et surface intérieur absorbant et qui est coupler avec extérieur par les passage (40, 42).

18. La méthode de contrôle du processus de réfrigeration qui utilise la tomographie a rayon X pour la visualisation de changement d'êtat par detection de changement de densité.

Fig. 1

*Fig. 2*

Fig. 3

36  31                     29      9      22    21   20      7

33                                                23

Fig. 4

0 108 112

*Fig. 5*

0 108 112

*Fig. 6*

0 108 112

Fig. 7